# EUROPEAN PATENT SPECIFICATION

(11) **EP 1 873 152 B1**
(45) Date of publication and mention of the grant of the patent: **15.10.2008**
(21) Application number: 06013134.9
(22) Date of filing: 26.06.2006
(51) Int. Cl.: C07D 403/12

(54) **Novel purification process of moxonidine**
Reinigungsverfahren von Moxonidine
Procédé de purification de moxonidine

(43) Date of publication of application: 02.01.2008
(73) Proprietor: CHEMAGIS LTD., 51200 Bnei-Brak (IL)
(72) Inventor: Naddaka, Vladimir, 71338 Lod (IL); Klopfer, Eyal, 64955 Tel Aviv (IL); Saeed, Shady, 33266 Haifa (IL); Arad, Oded, 76303 Rechovot (IL); Kaspi, Joseph, 53201 Givatayim (IL)
(74) Representative: Becker Kurig Straus

(56) References cited:
- US-A- 4 323 570
- CZESKIS, BORIS A.: "Synthesis of triple [14C]-labeled moxonidine" JOURNAL OF LABELLED COMPOUNDS & RADIOPHARMACEUTICALS , 47(10), 699-704 CODEN: JLCRD4; ISSN: 0362-4803, 2004, XP002394964
- "Impurities: Guideline for Residual Solvents, Q3C(R3)" 12 September 2002 (2002-09-12), INTERNATIONAL CONFERENCE ON HARMONISATION OF TECHNICAL REQUIREMENTS FOR REGISTRATION OF PHARMACEUTICALS FOR HUMAN USE , XP002394984 Tables 1-3, pages 5-7. Appendix 1, pages 9-12

## Description

### FIELD OF THE INVENTION

The present invention relates to a novel purification process of the drug Moxonidine.

### BACKGROUND OF THE INVENTION

Moxonidine (4-chloro-5-(imidazoline-2-ylamino)-6-methoxy-2-methylpyrimidine), has the structural formula (I) below and is used as an antihypertensive drug.

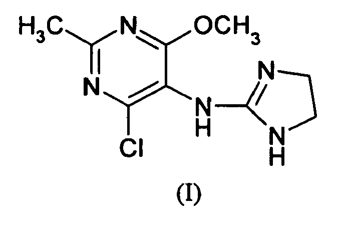

Moxonidine was approved for use in Germany in 1991 and is currently commercially available in Europe, e.g., in Germany, Austria and in the UK.

U.S. patent No. 4,323,570 (hereinafter the '570 patent) describes a method of preparing Moxonidine (I) by reacting 4,6-dichloro-2-methyl-5-(1-acetyl-2-imidazolin-2-yl)-aminopyrimidine (hereinafter DMAIA) with about 2 equivalents of sodium methoxide in methanol under reflux. It is stated in example 3 of the '570 patent that Moxonidine is obtained by crystallization from nitromethane having a melting point of 217-219°C. The hydrochloride salt of Moxonidine is described in example 25, having a melting point of 189°C.

Czech patent No. 294649 (hereinafter the '649 patent) describes a method of preparing Moxonidine from DMAIA by methanolysis reaction using alkali metal carbonates e.g., potassium carbonate at 47-52°C or sodium bicarbonate at 65°C.

Nitromethane, the solvent from which moxonidine is recrystallized, is a hazardous and explosive solvent in addition to being toxic (the allowed limit of nitromethane in human drugs, according to ICH guideline is merely 50 ppm). It is incompatible with many commonly used industrial reagents e.g., amines, strong acids and strong bases. Therefore, the crystallization from nitromethane is not suitable for industrial implementation due to its hazardous nature. Its toxicity renders it not suitable for use in human drugs. Thus there is a need in the art for an alternative purification process of Moxonidine using safer and more environmentally friendly solvents.

### BRIEF SUMMARY OF THE INVENTION

While searching for an improved process for preparing and purifying Moxonidine, the inventors of the present invention have reproduced example 3 of the '570 patent and example 1 of the '649 patent and found that in both cases the crude Moxonidine contained substantial levels of the impurities 4,6-dimethoxy-2-methyl-5-(2-imidazolin-2-yl)-aminopyrimidine of formula (II) and 4,6-dichloro-2-methyl-5-(2-imidazolin-2-yl)-aminopyrimidine of formula (III) as described in reference examples 1 and 2 respectively.

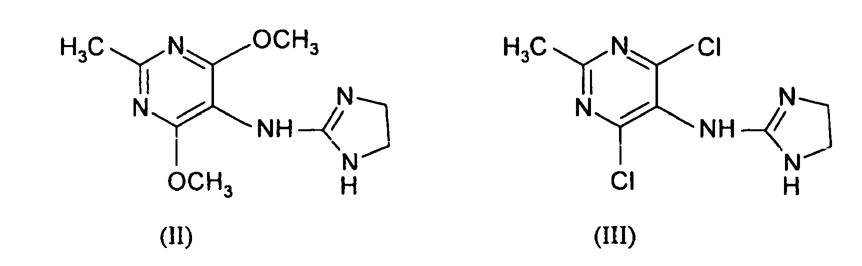

It is apparent to those skilled in the art that purifying crude Moxonidine from these impurities, namely compounds (II) and (III), may not be an easy task to achieve, and the purified Moxonidine is liable to be obtained in a relatively low yield.

According to the present invention, in a search for environmentally friendly non-toxic and non-hazardous solvents that can efficiently replace the use of nitromethane for purification of crude Moxonidine and significantly reduce the content of the impurities, compounds (II) and (III), it has been discovered that certain ICH class 3 solvents or selected class 2 solvents, which are safer and more environmentally friendly than nitromethane, may be used instead for preparing highly pure Moxonidine.

The inventors of the present invention have surprisingly discovered that not only it is possible to purify crude Moxonidine from high boiling point polar solvents and to obtain the crystallized Moxonidine in high quality and yield, but also the final product is obtained containing a negligible amount of the solvent.

According to the present invention, the process for preparing the purified Moxonidine comprises:
dissolving crude Moxonidine in the solvent optionally at elevated temperature;
allowing the mixture to cool sufficiently; and
collecting the crystals by filtration and drying.

According to a preferred embodiment of the present invention, the purified Moxonidine is obtained as described herein having residual solvent of less than 1000 ppm, preferably of less than 500 ppm and more preferably of less than 250 ppm.

According to an embodiment of the present invention, the purified Moxonidine is obtained as described herein having a purity of at least 98%, preferably in a purity equal to or greater than 99.5%, and more preferably in a purity equal to or greater than 99.8%.

By crystallizing crude Moxonidine from different solvents, e.g., DMSO, or by slurrying crude Moxonidine in other solvents, e.g., acetone, a crystalline solid comprising Moxonidine form I is obtained.

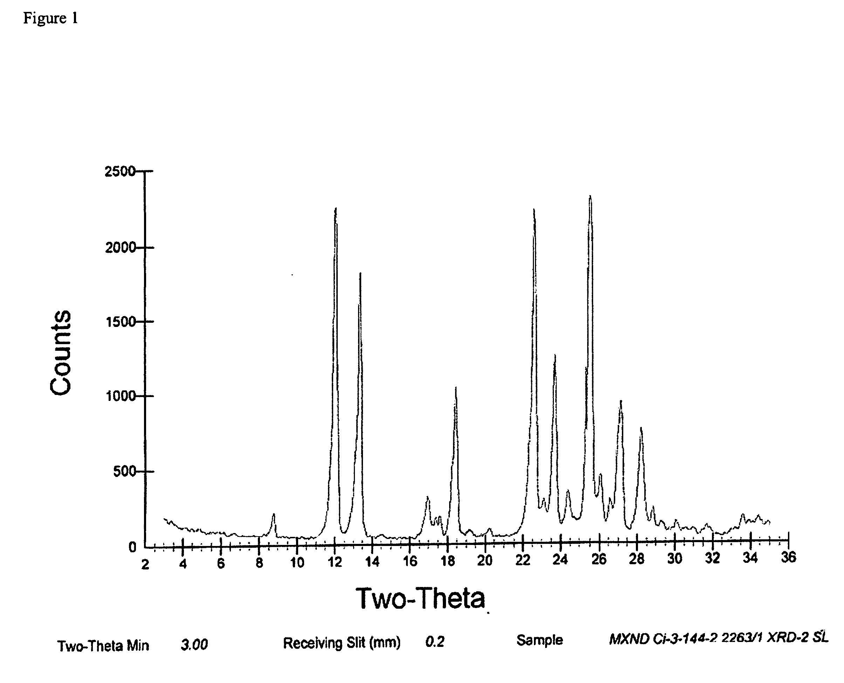

The crystalline Moxonidine form I produces a unique X-ray powder diffraction pattern (figure 1). The strong diffraction peaks at 12.1, 13.4, 18.4, 22.6, 23.7, 25.5, 27.1 and 28.2±0.2 degrees 2θ are most characteristic of this form.

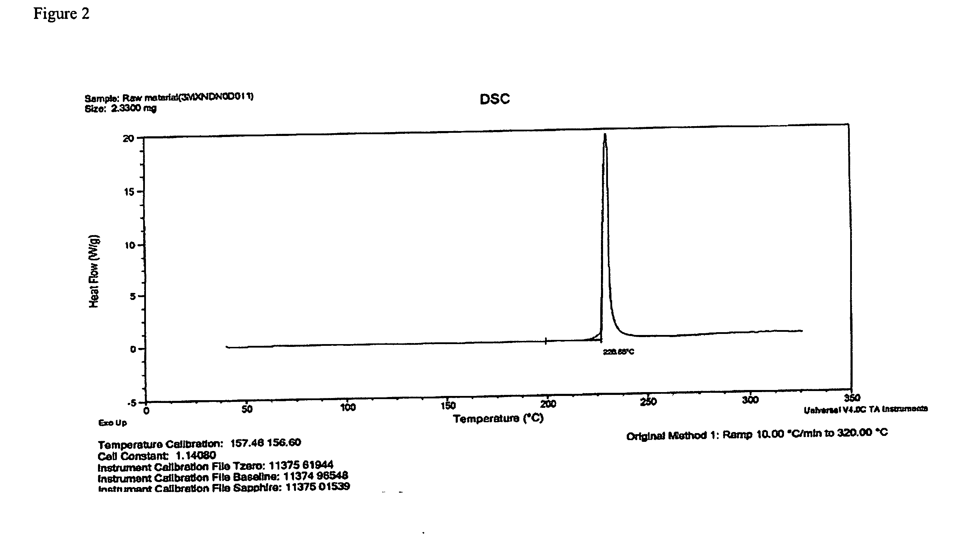

A characteristic DSC curve of the crystalline Moxonidine form I, which contains an exothermic peak at about 227°C, is depicted in figure 2.

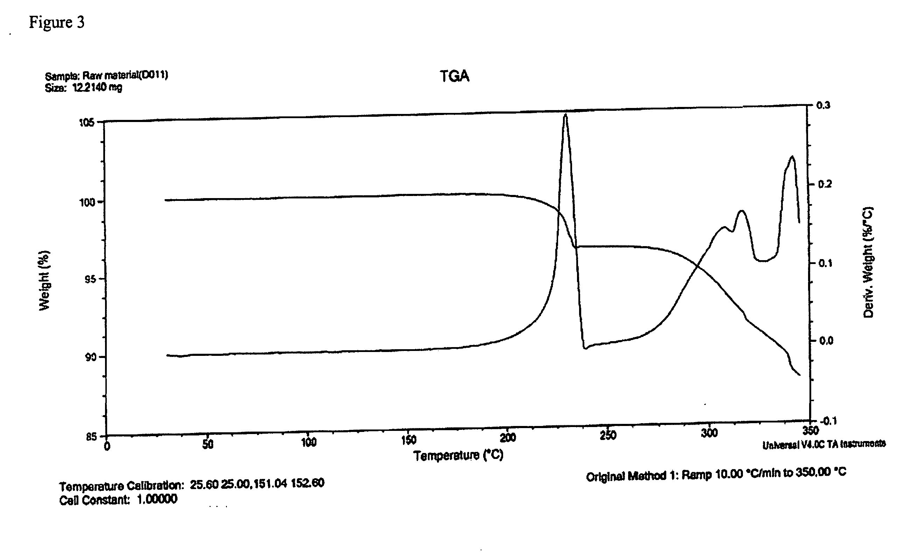

The characteristic TGA curve of the crystalline Moxonidine form I is depicted in figure 3.

### BRIEF DESCRIPTION OF FIGURES

Figure 1 - depicts the X-ray powder diffraction pattern of the crystalline Moxonidine form I
Figure 2 - depicts the DSC curve of the crystalline Moxonidine form I
Figure 3 - depicts the TGA curve of the crystalline Moxonidine form I

### DESCRIPTION OF THE PREFERRED EMBODIMENTS

While searching for an improved process for preparing and purifying Moxonidine, the inventors of the present invention have reproduced example 3 of the '570 patent and example 1 of the '649 patent and found that in both cases the crude Moxonidine contained substantial levels of the impurities 4,6-dimethoxy-2-methyl-5-(2-imidazolin-2-yl)-aminopyrimidine of formula (II) and 4,6-dichloro-2-methyl-5-(2-imidazolin-2-yl)-aminopyrimidine of formula (III) as described in reference examples 1 and 2 respectively.

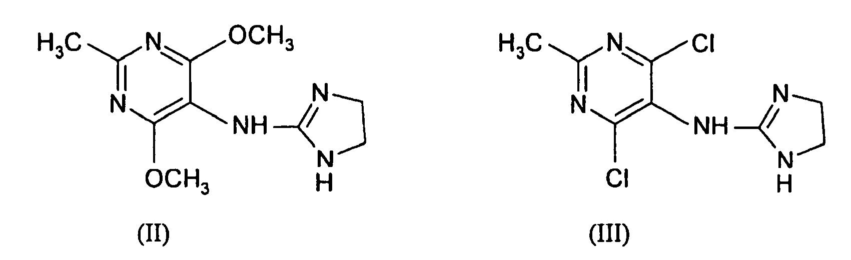

It is apparent to those skilled in the art that purifying crude Moxonidine from these impurities, namely compounds (II) and (III), may not be an easy task to achieve, and the purified Moxonidine is liable to be obtained in a relatively low yield.

Therefore, there is a need in the art for an improved process for producing and purifying Moxonidine in high yield, wherein the content of theses impurities is minimal.

According to the present invention, in a search for environmentally friendly non-toxic and non-hazardous solvents that can efficiently replace the use of nitromethane for purification of crude Moxonidine and significantly reduce the content of the impurities, compounds (II) and (III), it has been discovered that certain ICH class 3 solvents or selected class 2 solvents, which are safer and more environmentally friendly than nitromethane, may be used instead for preparing highly pure Moxonidine.
The inventors of the present invention have used the guidance "Q3C: Residual Solvents" published by the "International Conference on Harmonization of Technical Requirements of Registration of Pharmaceuticals for Human Use (ICH)". A copy of this guidance can be found in the US Federal register Volume 62, No. 247 (December 24, 1974) Docket 97D-0148. According to this guidance (Appendixes 5-7: toxicological data for class 1-3 solvents respectively), the use of industrial solvents in active pharmaceutical ingredients is restricted according to their toxicity and safety features. The industrial solvents are divided into three main classes:
Class 1: Solvents to be avoided. These are solvents that should not be employed in the manufacture of drug substances or drug products because of their unacceptable toxicity or their deleterious environmental effect. Solvents that belong to this class are: benzene, carbon tetrachloride, 1,2-dichloroethane and others.
Class 2: Solvents to be monitored. These are solvents that should be limited in pharmaceutical products because of their inherent toxicity. Important industrial solvents that belong to this class are chlorinated solvents such as chloroform dichloromethane, hydrocarbons such as hexane and aromatic solvents such as toluene.
Class 3: Solvents that are regarded as less toxic and of lower risk to human health. Important industrial solvents that belong to this class are certain ketones, esters, alcohols and others.

According to the present invention, not all the solvents in class 3 and class 2 produce equally good results with respect to the solubility and purification ability. Many of these solvents are unsuitable for purifying Moxonidine because they do not dissolve the material or the amount of boiling solvent needed to dissolve Moxonidine is so big it exceeds any reasonable, economical rationale. Some solvents dissolve Moxonidine and it stays dissolved even upon cooling. Other solvents are unsuitable for purifying Moxonidine because although they dissolve Moxonidine well, preferably at elevated temperature, and the material precipitates upon cooling, the Moxonidine thus obtained is hardly purified, if at all. It was found that only a limited number of class 3 and class 2 solvents are suitable for purifying moxonidine. These solvents are highly polar ones: dimethyl sulfoxide (DMSO), N,N-dimethylformamide (DMF), N,N-dimethylacetamide (DMA), and N-methyl-2-pyrrolidone (NMP). These solvents gave highly pure Moxonidine in high yield.

These polar solvents have high boiling points, e.g., the boiling point of DMSO is 189°C, that of DMA is 165°C and for NMP the value is 202°C. Therefore it is usually difficult to eliminate the solvent from the final product, which tends to contain substantial amount of the solvent.

The inventors of the present invention have surprisingly discovered that not only it is possible to purify crude Moxonidine from high boiling point polar solvents and to obtain the crystallized Moxonidine in high quality and yield, but also the final product is obtained containing a negligible amount of the solvent.

While it was discovered that some polar solvents can purify Moxonidine efficiently, this was right mainly for removal of the impurity having formula III. Low values of the compound of formula II resisted quite successfully their removal.

The inventors of the present invention have surprisingly found that small amounts of acid, such as acetic acid can reduce significantly the levels of compound II in Moxonidine.

Thus, in one embodiment, the present invention provides a process for preparing the crystalline Moxonidine by crystallization from the above solvents for which the permitted level in human drugs is 500 ppm or higher.

According to the present invention, high boiling point polar solvents, e.g., dimethyl sulfoxide (DMSO), N,N-dimethylformamide (DMF), N,N-dimethylacetamide (DMA), N-methyl-2-pyrrolidone (NMP), or a mixture thereof are most suitable for purifying Moxonidine because while crystallizing from these solvents, the content of the impurity compound (III) is significantly reduced.

In another embodiment, Moxonodine may be crystallized from a mixture of a high boiling point solvent e.g., dimethyl sulfoxide (DMSO) and an acid, wherein a suitable acid can be either an inorganic acid or an organic acid, e.g., formic acid, acetic acid or propionic acid. The use of a mixture of e.g., DMSO and acetic acid is advantageous in significantly reducing the content of compound (II), as depicted in tables 2 and 4 of the present invention.

According to another preferred embodiment of the present invention, the purified Moxonidine is obtained as described herein having residual solvent of less than 1000 ppm, preferably of less than 500 ppm and more preferably of less than 250 ppm.

According to yet another embodiment of the present invention, the purified Moxonidine is obtained as described herein having a purity of at least 98%, preferably in a purity equal to or greater than 99.5%, and more preferably in a purity equal to or greater than 99.8%.

By crystallizing crude Moxonidine from different solvents, e.g., DMSO, or by slurrying crude Moxonidine in other solvents, e.g., acetone, a crystalline solid comprising Moxonidine form I is obtained.

The crystalline Moxonidine form I produces a unique X-ray powder diffraction pattern (figure 1 and table 1). The strong diffraction peaks at 12.1, 13.4, 18.4, 22.6, 23.7, 25.5, 27.1 and 28.2±0.2 degrees 2θ are most characteristic of this form.

**Table 1 - Crystalline Moxonidine form I - X-ray powder diffraction peak positions and intensities**

| Peak position 2θ degrees | Relative intensity I/I₀ | | Peak position 2θ degrees | Relative intensity I/I₀ |
|---|---|---|---|---|
| 8.8 | 4.6 | | 24.4 | 7.0 |
| 12.1 | 82.4 | | 25.5 | 100.0 |
| 13.4 | 49.6 | | 26.1 | 9.8 |
| 17.0 | 6.9 | | 26.6 | 5.8 |
| 17.4 | 3.4 | | 27.1 | 21.8 |
| 17.6 | 4.0 | | 28.2 | 17.5 |
| 18.4 | 24.9 | | 28.8 | 4.7 |
| 19.2 | 0.9 | | 29.3 | 1.9 |
| 20.3 | 1.3 | | 30.1 | 2.4 |
| 22.6 | 56.7 | | 31.7 | 1.4 |
| 23.1 | 5.9 | | 33.6 | 3.2 |
| 23.7 | 30.3 | | 34.4 | 3.0 |

The characteristic DSC curve of the crystalline Moxonidine form I, which contains an exothermic peak at about 227°C, is depicted in figure 2.

The characteristic TGA curve of the crystalline Moxonidine form I is depicted in figure 3.

The crystalline Moxonidine form I is prepared by slurrying in or crystallizing from a solvent selected from the group consisting of water, glycols wherein a preferred glycol is propylene glycol; polar solvents, wherein preferred polar solvents are dimethyl sulfoxide (DMSO), N,N-dimethylformamide (DMF), N,N-dimethylacetamide (DMA), and N-methyl-2-pyrrolidone (NMP); alcohols, wherein preferred alcohols are methanol, ethanol, 1-propanol, 2-propanol, 1-butanol, 2-butanol, 2-methyl-1-propanol, 3-methyl-1-propanol, and n-amyl-alcohol; R₁COOR₂ esters, while R₁=C₁-C₅ alkyl and R₂=C₁-C₅ alkyl, wherein preferred esters are ethyl formate, methyl acetate, ethyl acetate, n-propyl acetate, isopropyl acetate, n-butyl acetate, isobutyl acetate, and tert-butyl acetate; C₁-C₆ ketones, wherein preferred ketones are acetone, methyl ethyl ketone (MEK), diethyl ketone, methyl propyl ketone, and methyl isobutyl ketone (MIBK); halogenated solvents, wherein a preferred halogenated solvent is dichloromethane; ethers, wherein preferred ethers are diethyl ether, tert-butyl methyl ether, and tetrahydrofuran (THF); aromatic solvents, wherein preferred aromatic solvents are anisole, cumene and toluene; hydrocarbons, wherein preferred hydrocarbons are pentane, hexane, heptane, cyclohexane and methylcyclohexane, and mixtures thereof.

### EXAMPLES

HPLC measurements of Moxonidine samples were performed using HPLC system, equipped with Phenomenex Luna 5µ C8(2) (4.6 x 250 mm) column, and a UV detector operated on 230 nm. Analyses were performed using the following mobile phase, at flow rate of 1.2 ml/minute. Eluent A: 10 mM pentanesulfonic acid, pH = 3.0 with H₂SO₄. Eluent B: acetonitrile. Gradient (A/B, v/v): 0 min (94/6), 4 min (94/6), 20 min (64/36), 50 min (64/36).

### Reference Example 1 - Preparation of Moxonidine by reaction of 4,6-dichloro-2-methyl-5-(1-acetyl-2-imidazolin-2-yl)-aminopyrimidine (DMAIA) with 2.02 equivalents of sodium methoxide at boiling temperature

4,6-dichloro-2-methyl-5-(1-acetyl-2-imidazolin-2-yl)-aminopyrimidine, (DMAIA), (10 g, 0.0347 mol) was mixed with a solution of sodium methoxide (3.78 g, 0.07 mol, 2.02 equiv.) in 35 ml of methanol and boiled for 2 hours. Then, water (100 ml) was added and the reaction mixture was cooled to ambient temperature. A colorless product was collected by filtration, washed with water and dried at 50° C overnight to yield crude Moxonidine containing 6.5% of impurity (II) and 0.61 % of impurity (III).

### Reference Example 2 - Preparation of Moxonidine by reaction of DMAIA with 2 molar equivalents of potassium carbonate at 47-52° C.

Potassium carbonate (9.6 g, 0.0694 mol, 2 molar equiv.) was added to a suspension of DMAIA (10 g, 0.0347 mol) in methanol (80 ml) and the mixture was heated at 47-52° C for 5 hours. Then, the reaction mixture was cooled to ambient temperature and acetic acid (10 ml) and water (70 ml) were added. After stirring for half an hour, 25% solution of ammonium hydroxide (10 ml) was added and the mixture was stirred for one hour. A colorless precipitate was collected by filtration, washed with water and dried at 50° C overnight to yield crude Moxonidine containing 0.07% of impurity (II) and 0.40% of impurity (III).

### Example 1- Preparation of crude Moxonidine by an improved process, as described in a copending application

A mixture of DMAIA (5 g, 0.0174 mol) and 85% potassium hydroxide powder (1.276 g, 0.019 mol, 1.1 equiv.) in methanol (40 ml) was stirred at ambient temperature for 30 hours. Then, water (50 ml) was added and the mixture was stirred for one hour. A colorless precipitate was collected by filtration, washed with water (3 x10 ml) and 2-propanol (3 x10 ml) and dried at 50° C overnight to give 3.5 g of crude Moxonidine in 83.5% yield, having a purity of 98.2% (by HPLC).

### Example 2 - Crystallization of crude Moxonidine from DMSO

Crude Moxonidine (2.0 g), having a purity of 99.12% and containing 0.03% of impurity (II) and 0.85% of impurity (III), was mixed with DMSO (10 ml) and heated to a temperature of about 90°C to obtain a clear solution. The solution was cooled to ambient temperature and the thus formed crystals were obtained by filtration and washed with cold 2-propanol and dried under reduced pressure. 1.7 g of crystallized Moxonidine were obtained in 85% yield having a purity by HPLC of 99.91%, containing 0.02% of impurity (II) and 0.06% of impurity (III), and containing 218 ppm of residual DMSO. (The allowed residual amount of DMSO is 5000 ppm)

### Examples 3-6 - Crystallization of crude Moxonidine, containing different amounts of impurities, from DMSO

Crude Moxonidine samples (2.0 g), having different purities and containing different amounts of impurity (II) and (III), wherein the content of compound (III) was significantly higher (except for in example 3), were crystallized from DMSO and the results are summarized in table 2.

**Table 2 - Crystallization of crude Moxonidine with high content of impurity (III) from DMSO**

| Ex No. | Purity of crude, % | % (w/w) of (II) | % (w/w) of (III) | DMSO, vol. per weight ml/g | Yield of cryst, % | Purity of cryst, % | % (w/w) of (II) | % (w/w) of (III) |
|---|---|---|---|---|---|---|---|---|
| 3 | 94.41 | 4.64 | 0.95 | 6 | 80 | 93.66 | 6.28 | 0.06 |
| 4 | 99.13 | 0.14 | 0.73 | 5 | 81 | 99.70 | 0.16 | 0.14 |
| 5 | 98.34 | 0.09 | 1.57 | 5 | 81 | 99.71 | 0.09 | 0.20 |
| 6 | 98.89 | 0.07 | 1.04 | 5 | 82 | 99.84 | 0.07 | 0.09 |

### Examples 7-9 - Crystallization of Moxonidine from high boiling point polar solvents

Crude Moxonidine (5.0 g), having a purity of 99.12% (w/w), and containing 0.03% (w/w) of compound (II) and 0.85% (w/w) of compound (III), was mixed with the solvent, and heated to 90-100°C to obtain a clear solution. The solution was cooled to ambient temperature and the thus formed crystals were obtained by filtration and washed with cold 2-propanol and dried under reduced pressure. Crystalline Moxonidine was obtained in different yields.

**Table 3 - Crystallization of crude Moxonidine from high boiling point solvents**

| Ex No. | Solvent | Allowed level, ppm | Vol. per weight ml/g | Yield, % | Product's purity, % | % of (II) | % of (III) |
|---|---|---|---|---|---|---|---|
| 7 | DMF | 880 | 7 | 82 | 99.69 | 0.03 | 0.28 |
| 8 | DMA | 1090 | 7 | 58 | 99.84 | 0.04 | 0.12 |
| 9 | NMP | 4840 | 4 | 68 | 99.75 | 0.03 | 0.22 |

### Example 10-12 - Crystallization of crude Moxonidine from mixtures of high boiling point solvent and acetic acid

Crude Moxonidine containing different amounts of the impurities compound (II) and compound (III) was crystallized from mixtures of high boiling point solvent and acetic acid, and the results are summarized in table 4.

**Table 4 - Crystallization of crude Moxonidine from mixtures of high boiling point solvent and acetic acid (AA).**

| Ex No. | Solvent | Vol. per weight ml/g | Purity of the crude % (w/w) | % (w/w) of (II) | % (w/w) of (III) | Yield % | Purity of crystallized Moxonidine % (w/w) | % (w/w) of (II) | % (w/w) of (III) |
|---|---|---|---|---|---|---|---|---|---|
| 10 | DMSO + AA | 5.5 + 0.1 | 99.30 | 0.50 | 0.20 | 60 | 99.77 | 0.21 | 0.02 |
| 11 | DMSO + AA | 5.5 + 0.1 | 98.04 | 0.92 | 1.04 | 62 | 99.31 | 0.44 | 0.25 |
| 12 | DMA + AA | 7 + 0.1 | 99.09 | 0.57 | 0.34 | 68 | 99.75 | 0.14 | 0.11 |

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A process for purifying Moxonidine by crystallization from a solvent which is a high boiling point polar solvent selected from the group consisting of N,N-dimethylacetamide (DMA), N,N-dimethylformamide (DMF), dimethyl sulfoxide (DMSO), N-methyl-2-pyrrolidone (NMP), or a mixture thereof, comprising:
dissolving crude Moxonidine in the solvent optionally at elevated temperature;
allowing the mixture to cool sufficiently; and
collecting the crystals by filtration and drying.

2. The process of claim 1, wherein the solvent is DMSO.

3. The process of claim 1, wherein an acid is added to the solvent.

4. The process of claim 3, wherein the acid is selected from the group consisting of formic acid, acetic acid and propionic acid.

5. The process of claim 4, wherein the acid is acetic acid.

6. The process of claim 5, wherein the amount of acetic acid is about 0.1 ml per 5.5 ml of dimethyl sulfoxide (DMSO).

7. The process of claim 5, wherein the amount of acetic acid is about 0.1 ml per 7 ml of N,N-dimethylacetamide (DMA).

8. The process of claim 2, wherein the crystallized Moxonidine, obtained by crystallization from DMSO, contains less than 1000 ppm residual solvent.

9. The process of claim 2, wherein the crystallized Moxonidine, obtained by crystallization from DMSO, contains less than 500 ppm residual solvent.

10. The process of claim 2, wherein the crystallized Moxonidine, obtained by crystallization from DMSO, contains less than 250 ppm residual solvent.

11. The process of claim 2, wherein the crystallized Moxonidine, is obtained having a purity equal to or greater than 99.5%.

## Patentansprüche

1. Verfahren zum Reinigen von Moxonidin durch Kristallisation aus einem Lösungsmittel, welches ein polares Lösungsmittel mit hohem Siedepunkt ist, ausgewählt aus der Gruppe bestehend aus N,N-Dimethylacetamid (DMA), N,N-Dimethylformamid (DMF), Dimethyl-Sulfoxide (DMSO), N-Methyl-2-pyrrolidon (NMP) oder einem Gemisch daraus, umfassend:
- Lösen von Roh-Moxonidin in dem Lösungsmittel, optional bei erhöhter Temperatur;
- Ermöglichen, dass das Gemisch ausreichend abkühlt; und
- Sammeln der Kristalle durch Filtrieren und Trocknen.

2. Verfahren nach Anspruch 1, wobei das Lösungsmittel DMSO ist.

3. Verfahren nach Anspruch 1, wobei dem Lösungsmittel eine Säure hinzugefügt wird.

4. Verfahren nach Anspruch 3, wobei die Säure ausgewählt ist aus der Gruppe, bestehend aus Ameisensäure, Essigsäure und Propionsäure.

5. Verfahren nach Anspruch 4, wobei die Säure Essigsäure ist.

6. Verfahren nach Anspruch 5, wobei die Menge an Essigsäure ungefähr 0,1 ml pro 5,5 ml Dimethyl-Sulfoxid (DMSO) ist.

7. Verfahren nach Anspruch 5, wobei die Menge an Essigsäure ungefähr 0,1 ml pro 7 ml von N, N-Dimethylacetamid (DMA) ist.

8. Verfahren nach Anspruch 2, wobei das kristallisierte Moxonidin, welches durch Kristallisation aus DMSO erhalten wird, weniger als 1000 ppm Rest-Lösungsmittel enthält.

9. Verfahren nach Anspruch 2, wobei das kristallisierte Moxonidin, welches durch Kristallisation aus DMSO erhalten wird, weniger als 500 ppm Rest-Lösungsmittel enthält.

10. Verfahren nach Anspruch 2, wobei das kristallisierte Moxonidin, welches durch Kristallisation aus DMSO erhalten wird, weniger als 250 ppm Rest-Lösungsmittel enthält.

11. Verfahren nach Anspruch 2, wobei das kristallisierte Moxonidin mit einer Reinheit von gleich oder größer als 99,5 % erhalten wird.

## Revendications

1. Procédé de purification de moxonidine par cristallisation à partir d'un solvant qui est un solvant polaire ayant un point d'ébullition élevé choisi dans le groupe constitué par le N,N-diméthylacétamide (DMA), le N,N-diméthylformamide (DMF), le diméthylsulfoxyde (DMSO), la N-méthyl-2-pyrrolidone (NMP), ou un mélange de ceux-ci, comprenant :
la dissolution de moxonidine brute dans le solvant, éventuellement à une température élevée ;
le refroidissement du mélange de manière suffisante ; et
le recueil des cristaux par filtration et séchage.

2. Procédé selon la revendication 1, dans lequel le solvant est le DMSO.

3. Procédé selon la revendication 1, dans lequel un acide est ajouté au solvant.

4. Procédé selon la revendication 3, dans lequel l'acide est choisi dans le groupe constitué par l'acide formique, l'acide acétique et l'acide propionique.

5. Procédé selon la revendication 4, dans lequel l'acide est l'acide acétique.

6. Procédé selon la revendication 5, dans lequel la quantité d'acide acétique est d'environ 0,1 ml pour 5,5 ml de diméthylsulfoxyde (DMSO).

7. Procédé selon la revendication 5, dans lequel la quantité d'acide acétique est d'environ 0,1 ml pour 7 ml de N,N-diméthylacétamide (DMA).

8. Procédé selon la revendication 2, dans lequel la moxonidine cristallisée, obtenue par cristallisation à partir de DMSO, contient moins de 1000 ppm de solvant résiduaire.

9. Procédé selon la revendication 2, dans lequel la moxonidine cristallisée, obtenue par cristallisation à partir de DMSO, contient moins de 500 ppm de solvant résiduaire.

10. Procédé selon la revendication 2, dans lequel la moxonidine cristallisée, obtenue par cristallisation à partir de DMSO, contient moins de 250 ppm de solvant résiduaire.

11. Procédé selon la revendication 2, dans lequel la moxonidine cristallisée obtenue possède une pureté supérieure ou égale à 99,5 %.
